# EUROPEAN PATENT APPLICATION

(11) **EP 4 659 736 A1**
(43) Date of publication of application: **10.12.2025**
(21) Application number: 24750206.5
(22) Date of filing: 29.01.2024
(51) Int. Cl.: A61K 8/49, A61K 8/19, A61K 8/22, A61K 8/41, A61K 8/44, A61K 8/46, A61Q 5/04

(54) **HAIR-SHAPE-IMPARTING METHOD**

(30) Priority: 30.01.2023 JP 2023011705
(71) Applicant: Kao Corporation, Chuo-ku Tokyo 103-8210 (JP)
(72) Inventor: SHIMAZU Ayako, Tokyo 131-8501 (JP); MATSUMOTO Jun, Matsumoto-shi, Nagano 390-0805 (JP)
(74) Representative: Hoffmann Eitle
(86) International application number: PCT/JP2024/002642
(87) International publication number: WO 2024/162257

(57) **Abstract**

Provided is a hair-shape-imparting method comprising in the below order: Step 1A: applying an A1 agent comprising a component (a1) reducing agent and having pH of from 8 to 11 to hair, and applying a B1 agent comprising 0.03 to 0.3% by mass of a component (b1) melanin precursor and a component (b2) alkali agent and having pH of from 8 to 11 to the hair, or Step 1B: applying an A2 agent comprising a component (a1) reducing agent and 0.03 to 0.3% by mass of a component (a2) melanin precursor and having pH of from 8 to 11 to hair; and Step 2: imparting a shape to the hair, the method further comprising in the below order: Step 3: applying a C agent comprising an oxidizing agent to the hair; and Step 4: rinsing the hair.

## Description

### Field of the Invention

The present invention relates to a hair shaping method.

### Background of the Invention

A perming technique has heretofore been known which involves cleaving a disulfide bond of keratin constituting hair using a reducing agent such as thioglycolic acid, imparting a straight or a curled shape to the hair, and fixing the shape with hydrogen peroxide water. A problem of the conventional perming technique is to lose hair suppleness and resilience, and suppleness due to weakened hair physical properties associated with a series of chemical treatments.

For example, Chinese Patent Application Publication No. 111920696 (Patent Literature 1) discloses a method including blending a reducing agent solution with a preparation containing cystine and a predetermined melanin precursor, then coating hair with the blend in a predetermined temperature range, imparting any shape to the hair, uniformly coating the hair with an oxidizing agent solution after a lapse of a predetermined time, washing the hair after a lapse of a predetermined time, coating the hair with a preparation containing a predetermined melanin precursor, a buffer solution, and an oxidizing agent, and keeping the hair in a predetermined temperature range for a predetermined time, with an object to provide a method for using a hair dye which can perform hair dyeing and permanent at the same time using a safe and nontoxic predetermined melanin precursor, improves color robustness, can shorten a hair dyeing time, and reduces damage on hair.

Recent evolution of hair heating instruments such as hair irons facilitates imparting a straight or curled shape to hair in daily styling. However, such a shape imparted with a hair iron or the like generally returns to the original one by hair wash.

Accordingly, JP-A-2019-94328 (Patent Literature 2) discloses a method comprising the steps of: coating hair with a composition containing a predetermined melanin precursor; and imparting a shape to the hair in a predetermined range of a temperature, with an object to provide a hair treatment method which imparts a shape to hair without chemically deteriorating hair protein, and can maintain the shape even after hair wash.

### Citation List

### Patent Literature

Patent Literature 1: Chinese Patent Publication No. 111920696
Patent Literature 2: Japanese Patent Publication No. 2019-94328

### Summary of the Invention

The present invention relates to the following [1].
[1] A hair-shape-imparting method comprising the below steps in the below order:
   Step 1A: applying an A1 agent comprising the following component (a1):
      (a1) a reducing agent
      and having pH of from 8 to 11 to hair, and
   then applying a B1 agent comprising the following component (b1) and component (b2):
      (b1) 0.03 to 0.3% by mass of a compound of the following formula (1) or a salt thereof: 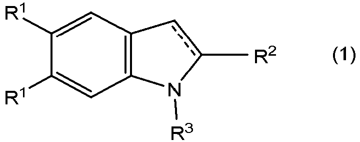 wherein the dashed line represents the presence or absence of a π bond; R¹ represents a hydroxy group or an acetoxy group; R² represents a hydrogen atom or -COOR, wherein R represents a hydrogen atom, a methyl group, or an ethyl group; and R³ represents a hydrogen atom, an acetyl group, a methyl group, or an ethyl group, and
      (b2) an alkali agent
      and having pH of from 8 to 11 to the hair, or
   Step 1B: applying an A2 agent comprising the following component (a1) and (a2):
      (a1) a reducing agent, and
      (a2) 0.03 to 0.3% by mass of a compound of the following formula (1) or a salt thereof: 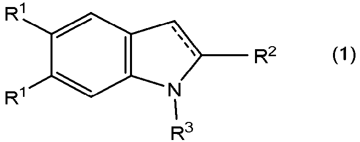 wherein the dashed line represents the presence or absence of a π bond; R¹ represents a hydroxy group or an acetoxy group; R² represents a hydrogen atom or -COOR, wherein R represents a hydrogen atom, a methyl group, or an ethyl group; and R³ represents a hydrogen atom, an acetyl group, a methyl group, or an ethyl group and having pH of from 8 to 11 to hair; and
   Step 2: imparting a shape to the hair,
   the method further comprising in the below order:
      Step 3: applying a C agent comprising an oxidizing agent to the hair; and
      Step 4: rinsing the hair.

### Detailed Description of the Invention

However, the method described in Patent Literature 1 disadvantageously cannot perform only the imparting of a shape to hair if hair dyeing is not desired. The method described in Patent Literature 2 has room for improvement in hair wash fastness of an imparted hair shape.

The present invention relates to a hair-shape-imparting method which can impart a hair shape having sufficient hair wash fastness without changing a hair color, impairs neither hair suppleness nor resilience, and offers excellent manageable feeling of hair.

The present inventors completed the present invention by finding that the problems described above could be solved by a hair-shape-imparting method comprising in the below order the steps of: applying an A1 agent comprising a reducing agent to hair and then applying an A2 agent comprising a predetermined melanin precursor to the hair (step 1A), or applying a B1 agent comprising a reducing agent and a predetermined melanin precursor to hair (step 1B); and imparting a shape to the hair (step 2), the method further comprising in the below order the steps of: applying a C agent comprising an oxidizing agent to the hair (step 3); and rinsing the hair (step 4).

The present invention can provide a hair-shape-imparting method which can impart a hair shape having sufficient hair wash fastness without changing a hair color, impairs neither hair suppleness nor resilience, and offers excellent manageable feeling of hair.

### [Definition]

In the present specification, the phrase "not change a hair color" means that an absolute value of change in L value between before and after a hair treatment is small, and, specifically, can be evaluated by a method described in Examples.

In the present specification, the phrase "having sufficient hair wash fastness" means that a shape imparted to hair is maintained even if a hair wash treatment with a shampoo is repeated several times, and, specifically, can be evaluated by a method described in Examples.

### [Hair-shape-imparting method]

The hair-shape-imparting method of the present invention (hereinafter, also simply referred to as the "method of the present invention") comprises in the below order:
Step 1A: applying an A1 agent comprising the following component (a1):
   (a1) a reducing agent
   and having pH of from 8 to 11 to hair, and
then applying a B1 agent comprising the following component (b1) and component (b2):
   (b1) 0.03 to 0.3% by mass of a compound of the following formula (1) or a salt thereof: 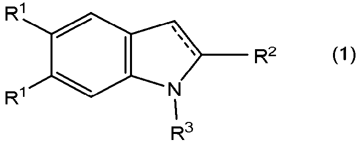 wherein the dashed line represents the presence or absence of a π bond; R¹ represents a hydroxy group or an acetoxy group; R² represents a hydrogen atom or -COOR (wherein R represents a hydrogen atom, a methyl group, or an ethyl group); and R³ represents a hydrogen atom, an acetyl group, a methyl group, or an ethyl group, and
   (b2) an alkali agent
   and having pH of from 8 to 11 to the hair, or
Step 1B: applying an A2 agent comprising the following component (a1) and (a2):
   (a1) a reducing agent, and
   (a2) 0.03 to 0.3% by mass of a compound of the following formula (1) or a salt thereof: 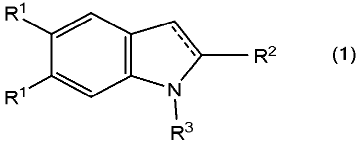 wherein the dashed line represents the presence or absence of a π bond; R¹ represents a hydroxy group or an acetoxy group; R² represents a hydrogen atom or -COOR (wherein R represents a hydrogen atom, a methyl group, or an ethyl group); and R³ represents a hydrogen atom, an acetyl group, a methyl group, or an ethyl group and having pH of from 8 to 11 to hair; and
Step 2: imparting a shape to the hair,
the method further comprising in the below order:
   Step 3: applying a C agent comprising an oxidizing agent to the hair; and
   Step 4: rinsing the hair.

The present invention, by having the configuration described above, can impart a hair shape having sufficient hair wash fastness without changing a hair color, impairs neither hair suppleness nor resilience, and offers excellent manageable feeling of hair. Specifically, the method of the present invention involves treating hair with the A1 agent comprising a reducing agent and then applying the B1 agent comprising a predetermined melanin precursor to the hair, or applying the A2 agent comprising a reducing agent and a predetermined melanin precursor to hair, and then impairs neither suppleness nor resilience and improves manageability, in the hair thus provided with the shape as compared with hair without the application of the predetermined melanin precursor. Although a detailed reason for obtaining such effects is not certain, it may be considered that the reducing agent cleaves a disulfide bond of keratin constituting hair, and the melanin precursor is contacted with this hair so that the melanin precursor penetrates the inside of the hair and then forms a polymer within the hair in a rinsing step, leading to improving manageability without impairing hair suppleness and resilience. Since a content of the predetermined melanin precursor in the B1 agent or the A2 agent falls within a specific range, a hair color is not changed between before and after the treatment.

### <Steps 1A and 1B>

The step 1A is the step of applying the A1 agent and the B1 agent mentioned later to hair. The step 1B is the step of applying the A2 agent mentioned later to hair. The method of the present invention preferably comprises the step 1A from the viewpoint of obtaining favorable hair suppleness and resilience, and manageable feeling, and preferably comprises the step 1B from the viewpoint of a convenient treatment.

### (A agent)

An A agent is an A1 agent comprising a component (a1) given below and having pH of from 8 to 11, or an A2 agent comprising a component (a1) and a component (a2) given below and having pH of from 8 to 11. Hereinafter, the A1 agent and the A2 agent are also referred to as the A agent.
(a1) A reducing agent, and
(a2) 0.03 to 0.3% by mass of a compound of the following formula (1) or a salt thereof: 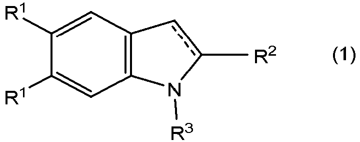 wherein the dashed line represents the presence or absence of a π bond; R¹ represents a hydroxy group or an acetoxy group; R² represents a hydrogen atom or -COOR (wherein R represents a hydrogen atom, a methyl group, or an ethyl group); and R³ represents a hydrogen atom, an acetyl group, a methyl group, or an ethyl group.

### <<Component (a1): reducing agent>>

The reducing agent can cleave a disulfide bond of keratin constituting hair. Examples of the reducing agent include thioglycolic acid and derivatives thereof, thiolactic acid and derivatives thereof, cysteine and derivatives thereof, and their salts. Specific examples of the reducing agent include thioglycolic acid, ammonium thioglycolate, thioglycolic acid glycerin ester, L-cysteine, D-cysteine, N-acetylcysteine, ammonium salts of these cysteines, ethanolamine salts such as monoethanolamine, diethanolamine, and triethanolamine, thioglyceryl alkyl ether such as ethoxyhydroxypropanethiol, methoxyethoxyhydroxypropanethiol, and isopropoxyethoxyhydroxypropanethiol, mercaptoethylpropanamide, and mercaptoethylgluconamide. Among them, the component (a1) preferably comprises one or more members selected from the group consisting of thioglycolic acid, ammonium thioglycolate, thioglycolic acid glycerin ester, L-cysteine, D-cysteine, N-acetylcysteine, and ammonium salts of these cysteines, and more preferably comprises one or more members selected from the group consisting of thioglycolic acid, ammonium thioglycolate, and thioglycolic acid glycerin ester.

A content of the reducing agent in the A agent is preferably 0.1% by mass or more, more preferably 1% by mass or more, further more preferably 2% by mass or more, even more preferably 5% by mass or more, and preferably 40% by mass or less, more preferably 20% by mass or less, further more preferably 15% by mass or less, even more preferably 10% by mass or less, from the viewpoint of being able to impart a hair shape having sufficient hair wash fastness, and obtaining favorable hair suppleness and resilience, and manageable feeling. The content of the reducing agent in the A agent is preferably from 0.1 to 40% by mass, more preferably from 1 to 20% by mass, further more preferably from 2 to 15% by mass, even more preferably from 5 to 10% by mass, from the viewpoint described above.

### <<Component (a2): melanin precursor>>

The component (a2) melanin precursor is an indole derivative or an indoline derivative, or a salt thereof, which is a compound of the formula (1). In the present invention, one or more thereof can be used (in combination).

Examples of the compound of the formula (1) include 5,6-dihydroxyindole, 5,6-dihydroxyindole-2-carboxylic acid, methyl 5,6-dihydroxyindole-2-carboxylate, ethyl 5,6-dihydroxyindole-2-carboxylate, N-methyl-5,6-dihydroxyindole, N-methyl-5,6-dihydroxyindole-2-carboxylic acid, N-ethyl-5,6-dihydroxyindole, N-ethyl-5,6-dihydroxyindole-2-carboxylic acid, N-acetyl-5,6-dihydroxyindole, N-acetyl-5,6-dihydroxyindole-2-carboxylic acid, 5-acetoxy-6-hydroxyindole, 5-acetoxy-6-hydroxyindole-2-carboxylic acid, 5,6-dihydroxyindoline, 5,6-dihydroxyindoline-2-carboxylic acid, methyl 5,6-dihydroxyindoline-2-carboxylate, ethyl 5,6-dihydroxyindoline-2-carboxylate, N-methyl-5,6-dihydroxyindoline, N-methyl-5,6-dihydroxyindoline-2-carboxylic acid, N-ethyl-5,6-dihydroxyindoline, N-ethyl-5,6-dihydroxyindoline-2-carboxylic acid, N-acetyl-5,6-dihydroxyindoline, N-acetyl-5,6-dihydroxyindoline-2-carboxylic acid, 5-acetoxy-6-hydroxyindoline, and 5-acetoxy-6-hydroxyindoline-2-carboxylic acid.

Examples of the salt of the compound of the formula (1) include hydrochloride, hydrobromide, sulfate, phosphate, acetate, propionate, lactate, and citrate of any of the compounds. Among them, hydrobromide of the compound is preferred from the viewpoint of availability.

When R² in the formula (1) is -COOH, examples of the salt of the compound of the formula (1) include carboxylate thereof (wherein R² is -COO⁻X⁺ (X⁺ is a cation such as an alkali metal ion (e.g., Na⁺ and K⁺), an alkaline earth metal ion (e.g., Ca⁺ and Mg⁺), or an ammonium ion)).

The component (a2) preferably comprises one or more members selected from the group consisting of 5,6-dihydroxyindole, 5,6-dihydroxyindoline, 5,6-dihydroxyindoline-2-carboxylic acid, and their salts, more preferably comprises one or more members selected from the group consisting of 5,6-dihydroxyindole, 5,6-dihydroxyindoline, 5,6-dihydroxyindole-2-carboxylic acid, and 5,6-dihydroxyindoline hydrobromide, further more preferably comprises one or more members selected from the group consisting of 5,6-dihydroxyindole and 5,6-dihydroxyindole-2-carboxylic acid, and even more preferably comprises 5,6-dihydroxyindole and 5,6-dihydroxyindole-2-carboxylic acid, from the viewpoint of being able to impart a hair shape having sufficient hair wash fastness, and obtaining favorable hair suppleness and resilience, and manageable feeling.

When the component (a2) comprises 5,6-dihydroxyindole and 5,6-dihydroxyindole-2-carboxylic acid, a molar ratio between the 5,6-dihydroxyindole and the 5,6-dihydroxyindole-2-carboxylic acid is preferably in the range of from 50:50 to 99:1, more preferably in the range of from 80:20 to 99:1, further more preferably in the range of from 85:15 to 95:5, from the viewpoint of being able to impart a hair shape having sufficient hair wash fastness, and obtaining favorable hair suppleness and resilience, and manageable feeling.

When the component (a2) comprises 5,6-dihydroxyindole and 5,6-dihydroxyindole-2-carboxylic acid, the molar ratio between the 5,6-dihydroxyindole and the 5,6-dihydroxyindole-2-carboxylic acid in the component (a2) can be quantified by reverse-phase HPLC.

When the A agent is the A2 agent comprising the component (a2), a content of the component (a2) in the A2 agent is from 0.03 to 0.3% by mass, preferably from 0.05 to 0.25% by mass, more preferably from 0.055 to 0.2% by mass, further more preferably from 0.06 to 0.1% by mass, even more preferably from 0.065 to 0.08% by mass, from the viewpoint of being able to impart a hair shape having sufficient hair wash fastness, and obtaining favorable hair suppleness and resilience, and manageable feeling.

In this context, when the A agent is the A2 agent, a treatment amount ratio between the component (a1) and component (a2) [(a1)/(a2)] per unit hair dry mass for applying the A2 agent to hair is preferably 20 or more, more preferably 65 or more, further more preferably 80 or more, from the viewpoint of not changing a hair color, and preferably 250 or less, more preferably 150 or less, further more preferably 100 or less, from the viewpoint of manageable feeling, supple and resilient feeling, and not changing a hair color. The ratio is preferably from 20 to 250, more preferably from 65 to 150, further more preferably from 80 to 100.

In this aspect, a ratio between an amount of the component (a1) blended and an amount of the component (a2) blended into the A2 agent is directly the same as the treatment amount ratio. Hence, the A2 agent can be prepared by blending these components so as to make a desired treatment amount ratio.

### <<pH>>

The pH of the A agent is from 8 to 11, more preferably from 8.5 to 10.5, from the viewpoint of being able to impart a hair shape having sufficient hair wash fastness, and obtaining favorable hair suppleness and resilience, and manageable feeling. The pH of the A agent can be adjusted by the addition of a known pH adjuster.

The pH is a value measured at 25°C and, specifically, can be measured by a method described in Examples.

The A agent can comprise, in addition to the reducing agent and the predetermined melanin precursor, components which are usually used in cosmetic compositions, for example, an aqueous medium such as water, a lower alcohol, low-molecular diol, or triol, a pH adjuster, a surfactant, an antioxidant, a viscosity adjuster, silicone, an aromatic alcohol, a coloring component other than the component (a2), a polymer, an oil agent, an anti-dandruff agent, a vitamin preparation, a germicide, an anti-inflammatory agent, an antiseptic, a chelating agent, a humectant, a pearlescent agent, a ceramide, a fragrance, and an ultraviolet absorber.

A formulation of the A agent is not particularly limited and may be any formulation, for example, a liquid, a foam, a paste, a cream, a solid, or a powder. The formulation of the A agent is preferably a liquid, a paste, or a cream from the viewpoint of application to hair by coating, immersion, or the like.

A method for preparing the A agent is not particularly limited. This part can be prepared, for example, by blending the component (a1), the component (a2), and other components optionally used and mixing these components using a known stirring apparatus or the like.

### (Application of A agent to hair)

The hair in applying the A agent thereto may be in a dry state or a wet state. A bath ratio (dry mass of the hair : mass of water) is preferably from 1:0.1 to 1:10, more preferably from 1:0.2 to 1:5, further more preferably from 1:0.3 to 1:3, from the viewpoint of being able to impart a hair shape having sufficient hair wash fastness, and obtaining favorable hair suppleness and resilience, and manageable feeling. The dry mass of the hair means a mass of hair cleansed with a shampoo, blow-dried with hot air of a dryer for 30 minutes, and left overnight or longer at a temperature of 25°C under a relative humidity of 65%.

A method for applying the A agent to hair may not be limited as long as the method can contact the A agent with the hair. Examples thereof include a method of coating the hair with the A agent and a method of immersing the hair in the A agent.

An amount of the A agent applied to the hair is a bath ratio (dry mass of the hair : mass of the A agent) of preferably from 1:0.2 to 1:20, more preferably from 1:0.5 to 1:10, further more preferably from 1:1 to 1:5, in view of balance between the imparting of a hair shape having sufficient hair wash fastness and economic performance.

The hair to which the A agent is to be applied is preferably hair of the head and can be at least a portion of the hair of the head.

A time for which the A agent is applied to the hair, i.e., the total of a time for which the hair is coated with or immersed in the A agent and a time for which the resultant is left, can be appropriately changed and is preferably 3 minutes or longer, more preferably 5 minutes or longer, further more preferably 10 minutes or longer, from the viewpoint of allowing the components of the A agent to penetrate the inside of hair, and preferably 90 minutes or shorter, more preferably 60 minutes or shorter, further more preferably 45 minutes or shorter, even more preferably 30 minutes or shorter, from the viewpoint of reducing burden on a practitioner. The time for which the A agent is applied to the hair is preferably from 3 to 90 minutes, more preferably from 5 to 60 minutes, further more preferably from 10 to 45 minutes, even more preferably from 10 to 30 minutes, from the viewpoint described above.

A temperature in applying the A agent to the hair is not particularly limited and is preferably 40°C or lower, more preferably 35°C or lower, from the viewpoint of the handleability of the A agent, and preferably 20°C or higher, more preferably 25°C or higher, from the viewpoint of being able to impart a hair shape having sufficient hair wash fastness, and obtaining favorable hair suppleness and resilience, and manageable feeling. The temperature in applying the A agent to the hair is preferably 20 to 40°C, more preferably 25 to 35°C, from the viewpoint described above.

The method of the present invention preferably comprises, after applying the A agent to hair and, in the case of the A1 agent, before applying the B1 agent mentioned later or in the case of the A2 agent, before applying the step 2 mentioned later, the step of rinsing the hair (hereinafter, also simply referred to as a "rinsing step ") from the viewpoint of removing components of the A agent which have not penetrated the inside of the hair. The rinsing step can be performed, for example, by washing off the A agent applied to the hair with water.

### (B1 agent)

The B1 agent comprises the following components (b1) and (b2) and has pH of from 8 to 11:
(b1) 0.03 to 0.3% by mass of a compound of the following formula (1) or a salt thereof: 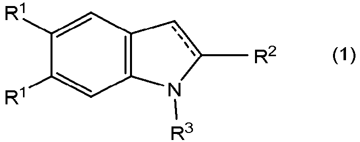
wherein the dashed line represents the presence or absence of a π bond; R¹ represents a hydroxy group or an acetoxy group; R² represents a hydrogen atom or -COOR (wherein R represents a hydrogen atom, a methyl group, or an ethyl group); and R³ represents a hydrogen atom, an acetyl group, a methyl group, or an ethyl group, and
(b2) an alkali agent.

### <<Component (b1): melanin precursor>>

The component (b1) melanin precursor is an indole derivative or an indoline derivative, or a salt thereof, which is a compound of the formula (1). In the present invention, one or more thereof can be used (in combination). Examples of the component (b1) can include the compounds listed in the component (a2) and their salts. The component (b1) preferably comprises one or more members selected from the group consisting of 5,6-dihydroxyindole, 5,6-dihydroxyindoline, 5,6-dihydroxyindoline-2-carboxylic acid, and their salts, more preferably comprises one or more members selected from the group consisting of 5,6-dihydroxyindole, 5,6-dihydroxyindoline, 5,6-dihydroxyindole-2-carboxylic acid, and 5,6-dihydroxyindoline hydrobromide, further more preferably comprises one or more members selected from the group consisting of 5,6-dihydroxyindole and 5,6-dihydroxyindole-2-carboxylic acid, and even more preferably comprises 5,6-dihydroxyindole and 5,6-dihydroxyindole-2-carboxylic acid, from the viewpoint of being able to impart a hair shape having sufficient hair wash fastness, and obtaining favorable hair suppleness and resilience, and manageable feeling.

When the component (b1) comprises 5,6-dihydroxyindole and 5,6-dihydroxyindole-2-carboxylic acid, a molar ratio between the 5,6-dihydroxyindole and the 5,6-dihydroxyindole-2-carboxylic acid is preferably in the range of from 50:50 to 99:1, more preferably in the range of from 80:20 to 99:1, further more preferably in the range of from 85:15 to 95:5, from the viewpoint of being able to impart a hair shape having sufficient hair wash fastness, and obtaining favorable hair suppleness and resilience, and manageable feeling.

When the component (b1) comprises 5,6-dihydroxyindole and 5,6-dihydroxyindole-2-carboxylic acid, the molar ratio between the 5,6-dihydroxyindole and the 5,6-dihydroxyindole-2-carboxylic acid in the component (b1) can be quantified by reverse-phase HPLC.

A content of the component (b1) in the B1 agent is from 0.03 to 0.3% by mass, preferably from 0.05 to 0.25% by mass, more preferably from 0.055 to 0.2% by mass, further more preferably from 0.06 to 0.15% by mass, even more preferably from 0.065 to 0.1% by mass, from the viewpoint of being able to impart a hair shape having sufficient hair wash fastness, and obtaining favorable hair suppleness and resilience, and manageable feeling.

### <<Component (b2): alkali agent>>

Examples of the alkali agent include: ammonia; alkanolamine such as mono-, di-, or trimethanolamine, and mono-, di-, or triethanolamine; alkylamine such as methylamine, dimethylamine, ethylamine, diethylamine, N-methylethylamine, propylamine, and butylamine; aralkylamine such as benzylamine; and inorganic alkali compounds such as sodium hydroxide, potassium hydroxide, sodium carbonate, sodium bicarbonate, ammonium carbonate, and ammonium bicarbonate, one or more of which can be used. The alkanolamine, the alkylamine, or the aralkylamine has preferably 10 or less, more preferably 8 or less carbon atoms from the viewpoint of water solubility.

Among them, the alkali agent preferably comprises one or more members selected from the group consisting of ammonia, alkanolamine, alkylamine, aralkylamine, sodium hydroxide, potassium hydroxide, sodium carbonate, sodium bicarbonate, ammonium carbonate, and ammonium bicarbonate, more preferably comprises one or more members selected from the group consisting of ammonia and alkanolamine, further more preferably comprises monoalkanolamine, and even more preferably comprises monoethanolamine, from the viewpoint of being able to impart a hair shape having sufficient hair wash fastness, and obtaining favorable hair suppleness and resilience, and manageable feeling.

A content of the component (b2) in the B1 agent is preferably from 0.1 to 10% by mass, more preferably from 0.5 to 8% by mass, further more preferably from 1 to 5% by mass, from the viewpoint of being able to impart a hair shape having sufficient hair wash fastness, and obtaining favorable hair suppleness and resilience, and manageable feeling.

### <<Component (b3): antioxidant>>

The B1 agent preferably further comprises (b3) an antioxidant from the viewpoint of improvement in stability of the component (b1).

Examples of the antioxidant include sulfurous acid, pyrosulfurous acid, hydrogen sulfite, sulfur dioxide, L-ascorbic acid, thioglycolic acid, L-cysteine, N-acetyl-L-cysteine, and their salts, and their derivatives. Examples of the salt include that of alkali metals such as sodium and potassium.

Among them, the antioxidant preferably comprises one or more members selected from the group consisting of L-ascorbic acid, sulfurous acid, pyrosulfurous acid, hydrogen sulfite, and their salts, ascorbic acid glucoside, and sulfur dioxide, more preferably comprises one or more members selected from the group consisting of L-ascorbic acid, sodium ascorbate, and sodium sulfite, and further more preferably comprises one or more members selected from the group consisting of L-ascorbic acid and sodium ascorbate.

When the B1 agent comprises the component (b3), a content of the component (b3) in the B1 agent is preferably 0.05% by mass or more, more preferably 0.1% by mass or more, further more preferably 0.3% by mass or more, and preferably 10% by mass or less, more preferably 5% by mass or less, further more preferably 2% by mass or less, from the viewpoint of improving the stability of the component (b1). The content is preferably from 0.05 to 10% by mass, more preferably from 0.1 to 5% by mass, further more preferably from 0.3 to 2% by mass, from the viewpoint described above.

### <<pH adjuster>>

The B1 agent preferably further comprises a pH adjuster from the viewpoint of adjusting pH to a desired range mentioned later, accelerating the polymerization reaction within hair of the component (b1), and fixing the component (b1) in the hair.

Since the B1 agent comprises the alkali agent as the component (b1), the pH adjuster for use in the B1 agent is preferably a protonating agent. The protonating agent may be any of monobasic acids and polybasic acids or may be any of organic acids (having 1 or more and 8 or less carbon atoms, except for ascorbic acid) and inorganic acids.

Examples of the protonating agent include one or more members selected from the group consisting of hydrochloric acid, sulfuric acid, phosphoric acid, formic acid, acetic acid, and citric acid. One or more members selected from the group consisting of phosphoric acid and citric acid is preferred.

When the B1 agent comprises the pH adjuster, a content of the pH adjuster in the B1 agent is preferably 0.05% by mass or more, more preferably 0.1% by mass or more, further more preferably 0.2% by mass or more, and preferably 5.0% by mass or less, more preferably 4.0% by mass or less, further more preferably 3.5% by mass or less, even more preferably 2.5% by mass or less, from the viewpoint of adjusting pH of the B1 agent to a range mentioned later.

### <<pH>>

The pH of the B1 agent is from 8 to 11, more preferably from 8.5 to 10.5, from the viewpoint of being able to impart a hair shape having sufficient hair wash fastness, and obtaining favorable hair suppleness and resilience, and manageable feeling.

The pH is a value measured at 25°C and, specifically, can be measured by a method described in Examples.

The B1 agent can comprise, in addition to the components (b1), (b2), and (b3), components which are usually used in cosmetic compositions, for example, an aqueous medium such as water, a lower alcohol, low-molecular diol, or triol, a higher alcohol, a surfactant such as a cationic surfactant or a nonionic surfactant, a viscosity adjuster, silicone, an aromatic alcohol, a polymer, an oil agent, an anti-dandruff agent, a vitamin preparation, a germicide, an anti-inflammatory agent, an antiseptic, a chelating agent, a humectant, a pearlescent agent, a ceramide, a fragrance, and an ultraviolet absorber.

A formulation of the B1 agent is not particularly limited and may be any formulation, for example, a liquid, a foam, a paste, a cream, a solid, or a powder. The formulation of the B1 agent is preferably a liquid, a paste, or a cream, more preferably a cream, from the viewpoint of application to hair by coating, immersion, or the like. A cream containing a cationic surfactant and a higher alcohol is further more preferred from the viewpoint of improving manageable feeling of hair.

The cationic surfactant is preferably one or more members selected from the group consisting of monoalkyl trimethylammonium chloride, dialkyl dimethylammonium chloride, monoalkyloxyalkyl trimethylammonium chloride, and monoalkyl trimethylammonium bromide, preferably one or more members selected from the group consisting of monoalkyl trimethylammonium chloride and monoalkyloxyalkyl trimethylammonium chloride, among them, more preferably one or more members selected from the group consisting of behenyl trimethylammonium chloride, stearyl trimethylammonium chloride, cetyl trimethylammonium chloride, lauryl trimethylammonium chloride, dialkyl (C₁₂ to C₁₈) dimethylammonium chloride, and octadecyloxypropyl trimethylammonium chloride.

When the B1 agent comprises the cationic surfactant, a content of the cationic surfactant in the B1 agent is preferably 0.1% by mass or more, more preferably 0.5% by mass or more, further more preferably 1.0% by mass or more, even more preferably 2.0% by mass or more, and preferably 10% by mass or less, more preferably 5% by mass or less, further more preferably 4% by mass or less.

A higher alcohol of the formula R⁴-OH (wherein R⁴ represents a linear or branched hydrocarbon group having 12 or more and 24 or less carbon atoms) can be used. R⁴ is preferably a linear or branched aliphatic hydrocarbon group having 12 or more and 24 or less carbon atoms, more preferably a linear or branched alkyl group having 12 or more and 24 or less carbon atoms or a linear or branched alkenyl group having 12 or more and 24 or less carbon atoms, further more preferably a linear alkyl group having 12 or more and 24 or less carbon atoms or a linear alkenyl group having 12 or more and 24 or less carbon atoms.

Examples of this higher alcohol include lauryl alcohol, myristyl alcohol, cetyl alcohol, stearyl alcohol, isostearyl alcohol, arachyl alcohol, behenyl alcohol, carnaubyl alcohol, and oleyl alcohol. One or more members selected from the group consisting of these alcohols can be used in combination.

When the B1 agent comprises the higher alcohol, a content thereof is preferably 2% by mass or more, more preferably 3% by mass or more, further more preferably 4% by mass or more, and preferably 10% by mass or less, more preferably 8% by mass or less, further more preferably 7% by mass or less, even more preferably 6% by mass or less.

The B1 agent preferably comprises a nonionic surfactant from the viewpoint of the stability of a preparation.

A polyoxyethylene-added nonionic surfactant can be used as the nonionic surfactant.

Specific examples of the polyoxyethylene-added nonionic surfactant include polyoxyethylene alkyl ether, polyoxyethylene alkenyl ether, polyoxyethylene monofatty acid ester, and polyoxyethylene sorbitan fatty acid ester. The alkyl group and the alkenyl group may be linear or branched. The fatty acid may have a saturated or unsaturated and linear or branched aliphatic chain.

The alkyl group, the alkenyl group, and the aliphatic chain in the fatty acid have preferably 8 or more, more preferably 10 or more, further more preferably 12 or more, and preferably 22 or less, more preferably 20 or less, further more preferably 18 or less carbon atoms from the viewpoint of suppressing the formation of aggregates.

When the B1 agent comprises the nonionic surfactant, a content thereof in the B1 agent is preferably 0.05% by mass or more, more preferably 0.3% by mass or more, further more preferably 0.5% by mass or more, even more preferably 0.7% by mass or more, and preferably 10% by mass or less, more preferably 7% by mass or less, further more preferably 5.0% by mass or less, even more preferably 3.0% by mass or less.

A method for preparing the B1 agent is not particularly limited. This part can be prepared, for example, by blending the component (b1), the component (b2), and other components optionally used and mixing these components using a known stirring apparatus or the like.

### (Application of B1 agent to hair)

The hair in applying the B1 agent thereto may be in a dry state or a wet state and is preferably in a wet state from the viewpoint of being able to impart a hair shape having sufficient hair wash fastness, and obtaining favorable hair suppleness and resilience, and manageable feeling.

A method for applying the B1 agent to hair may not be limited as long as the method can contact the B1 agent with the hair. The method for applying the A agent to hair mentioned above can be adopted.

An amount of the B1 agent applied to the hair is a bath ratio (dry mass of the hair : mass of the B1 agent) of preferably from 1:0.1 to 1:20, more preferably from 1:0.3 to 1:10, further more preferably from 1:0.5 to 1:5, in view of balance between the imparting of a hair shape having sufficient hair wash fastness and economic performance.

A treatment amount ratio between the reducing agent (a1) and the melanin precursor (b1) [(a1)/(b1)] per unit hair dry mass for applying the B1 agent to the hair is preferably 45 or more, more preferably 80 or more, further more preferably 150 or more, from the viewpoint of not changing a hair color, and preferably 460 or less, more preferably 300 or less, further more preferably 200 or less, from the viewpoint of manageable feeling, supple and resilient feeling, and not changing a hair color. The ratio is preferably from 45 to 460, more preferably from 80 to 300, further more preferably from 150 to 200.

In this aspect, the A1 agent and the B1 agent are respectively applied to hair at different steps. Therefore, the treatment amount ratio between the component (a1) and the component (b1) is determined depending on the content of the component (a1) in the A1 agent, the content of the component (b1) in the B1 agent, and the respective amounts of the A1 agent and the B1 agent applied to the hair, and can be easily adjusted.

A time for which the B1 agent is applied to the hair, i.e., the total of a time for which the hair is coated with or immersed in the B1 agent and a time for which the resultant is left, can be appropriately changed and is preferably 1 minute or longer, more preferably 3 minutes or longer, further more preferably 4 minutes or longer, from the viewpoint of allowing the components of the B1 agent to penetrate the inside of hair, and preferably 60 minutes or shorter, more preferably 30 minutes or shorter, further more preferably 15 minutes or shorter, from the viewpoint of reducing burden on a practitioner. The time for which the B1 agent is applied to the hair is preferably from 1 to 60 minutes, more preferably from 3 to 30 minutes, further more preferably from 4 to 15 minutes, from the viewpoint described above.

The method of the present invention preferably comprises, after applying the B1 agent to hair and before applying the step 2 mentioned later, a rinsing step of rinsing the hair from the viewpoint of removing components of the B1 agent which have not penetrated the inside of the hair.

Also, the method of the present invention can appropriately comprise, after the rinsing step, the step of drying the hair (hereinafter, also simply referred to as a "drying step"). The step of drying the hair is the step of reducing an amount of water in the hair. This step comprises, for example, performing one or more drying treatments selected from the group consisting of towel drying, blow (cold air or hot air) drying, and air drying in order to actively reduce the amount of water in the hair. Two or more of these drying treatments are preferably performed in combination.

### <Step of imparting shape to hair: step 2>

The method of the present invention further comprises the step of imparting a shape to the hair (step 2). The shape to be imparted to the hair can be a straight or curled shape and is preferably a straight shape.

Examples of a method for imparting a shape to the hair include a method of brushing the hair to impart a straight shape thereto, a method of holding the hair between the plates of a hair iron and imparting a straight shape to the hair while pressing the hair by sliding the hair iron from the root toward the tip of the hair, and a method of holding the hair between the plates of a hair iron and imparting a curled shape to the hair while wrapping the hair around the barrel of the hair iron.

The step 2 is preferably the step of drying the hair and heating the hair with a hair iron to impart a shape to the hair. The drying of the hair in this step is performed if the hair is not dry after the step 1 and before the step 2, and can be omitted when the drying step is performed in the step 1.

In the step 2, a method for drying the hair is not particularly limited, and the hair can be blow-dried with a hair dryer or the like.

A heating temperature of the hair with the hair iron is preferably 100°C or higher, more preferably 120°C or higher, further more preferably 140°C or higher, from the viewpoint of being able to impart a hair shape having sufficient hair wash fastness, and obtaining favorable hair suppleness and resilience, and manageable feeling, and preferably 220°C or lower, more preferably 210°C or lower, from the viewpoint of suppressing hair damage. The heating temperature of the hair is preferably from 100 to 220°C, more preferably from 120 to 220°C, further more preferably from 140 to 210°C, from the viewpoint described above.

### <Step of applying C agent to hair: step 3>

### (C agent)

The C agent comprises an oxidizing agent. Examples of the oxidizing agent include potassium bromate, sodium bromate, sodium perborate, and hydrogen peroxide. One or more of these oxidizing agents can be used (in combination). The oxidizing agent preferably comprises one or more members selected from the group consisting of sodium bromate and hydrogen peroxide, and is more preferably sodium bromate, from the viewpoint of being able to impart a hair shape having sufficient hair wash fastness, and obtaining favorable hair suppleness and resilience, and manageable feeling.

A content of the oxidizing agent in the C agent is preferably from 1 to 20% by mass, more preferably from 2 to 10% by mass, further more preferably from 3 to 8% by mass, from the viewpoint of sufficiently re-forming a disulfide bond of keratin constituting hair.

The C agent can comprise, in addition to the oxidizing agent, components which are usually used in cosmetic compositions, for example, an aqueous medium such as a lower alcohol, low-molecular diol, or triol, a pH adjuster, a surfactant, a viscosity adjuster, silicone, an aromatic alcohol, a polymer, an oil agent, an anti-dandruff agent, a vitamin preparation, a germicide, an anti-inflammatory agent, an antiseptic, a chelating agent, a humectant, a pearlescent agent, a ceramide, a fragrance, and an ultraviolet absorber.

The C agent preferably contains water as a balance of the oxidizing agent and components which are optionally used and usually used in cosmetic compositions.

A method for preparing the C agent is not particularly limited. This part can be prepared, for example, by blending the oxidizing agent, water, and other components optionally used and mixing these components using a known stirring apparatus or the like.

### (Application of C agent to hair)

The hair in applying the C agent thereto may be in a dry state or a wet state and is preferably in a dry state from the viewpoint of being able to impart a hair shape having sufficient hair wash fastness, and obtaining favorable hair suppleness and resilience, and manageable feeling. The C agent is preferably applied to the hair after the step 2.

A method for applying the C agent to the hair may not be limited as long as the method can contact the C agent with the hair. The method for applying the A agent to hair mentioned above can be adopted.

An amount of the C agent applied to the hair is a bath ratio (dry mass of the hair : mass of the C agent) of preferably from 1:0.1 to 1:20, more preferably from 1:0.5 to 1:10, further more preferably from 1:1 to 1:5, in view of balance between the imparting of a hair shape having sufficient hair wash fastness and economic performance.

A time for which the C agent is applied to the hair, i.e., the total of a time for which the hair is coated with or immersed in the C agent and a time for which the resultant is left, can be appropriately changed and is preferably 3 minutes or longer, more preferably 5 minutes or longer, further more preferably 10 minutes or longer, from the viewpoint of allowing the components of the C agent to penetrate the inside of hair, and preferably 90 minutes or shorter, more preferably 60 minutes or shorter, further more preferably 45 minutes or shorter, even more preferably 30 minutes or shorter, from the viewpoint of reducing burden on a practitioner. The time for which the C agent is applied to the hair is preferably from 3 to 90 minutes, more preferably from 5 to 60 minutes, further more preferably from 10 to 45 minutes, even more preferably from 10 to 30 minutes, from the viewpoint described above.

### <Step of rinsing hair :step 4>

The method of the present invention comprises, after applying the C agent to the hair, the step of rinsing the hair as a step 4 from the viewpoint of removing components of the C agent which have not penetrated the inside of the hair.

A method for rinsing the hair is not particularly limited, and the hair can be rinsed using, for example, water.

The method of the present invention imparts a shape to hair after application of the A agent or the B1 agent, and maintains the shape imparted to the hair even when the hair is rinsed after application of the C agent, and can impart a hair shape having sufficient hair wash fastness.

The method of the present invention can impart a straight shape or a curled shape to hair by applying the A agent or the B1 agent to the hair and heating the hair with a hair iron. Also, by applying the C agent to the hair, the hair treated by the method of the present invention maintains the shape imparted to the hair, has sufficient hair wash fastness, and has excellent manageable feeling without changing a hair color and without impairing hair suppleness and resilience.

### [Kit for imparting a hair shape]

The present invention also provides a kit for imparting a hair shape, comprising:
an A1 agent comprising the following component (a1):
   (a1) a reducing agent
   and having pH of from 8 to 11;
a B1 agent comprising the following component (b1) and component (b2):
   (b1) 0.03 to 0.3% by mass of a compound of the following formula (1) or a salt thereof: 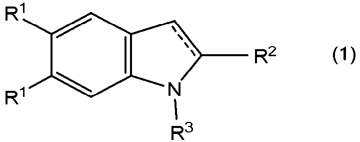 wherein the dashed line represents the presence or absence of a π bond; R¹ represents a hydroxy group or an acetoxy group; R² represents a hydrogen atom or -COOR (wherein R represents a hydrogen atom, a methyl group, or an ethyl group); and R³ represents a hydrogen atom, an acetyl group, a methyl group, or an ethyl group, and
   (b2) an alkali agent
   and having pH of from 8 to 11; and
a C agent comprising an oxidizing agent.

The A1 agent, the B1 agent, the C agent, and their preferred aspects are the same as those described in the hair-shape-imparting method.

The present invention also provides a kit for imparting a hair shape, comprising:
an A2 agent comprising the following components (a1) and (a2):
   (a1) a reducing agent, and
   (a2) 0.03 to 0.3% by mass of a compound of the following formula (1) or a salt thereof: 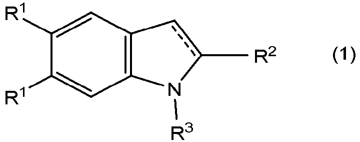 wherein the dashed line represents the presence or absence of a π bond; R¹ represents a hydroxy group or an acetoxy group; R² represents a hydrogen atom or -COOR (wherein R represents a hydrogen atom, a methyl group, or an ethyl group); and R³ represents a hydrogen atom, an acetyl group, a methyl group, or an ethyl group and having pH of from 8 to 11; and
a C agent comprising an oxidizing agent.

The A2 agent, the C agent, and their preferred forms are the same as those described in the hair-shape-imparting method.

Hereinafter, in relation to the embodiments mentioned above, the present invention further discloses the following.
<1> A hair-shape-imparting method comprising in the below order:
   Step 1A: applying an A1 agent comprising the following component (a1):
      (a1) a reducing agent
      and having pH of from 8 to 11 to hair, and
   then applying a B1 agent comprising the following component (b1) and component (b2):
      (b1) 0.03 to 0.3% by mass of a compound of the following formula (1) or a salt thereof: 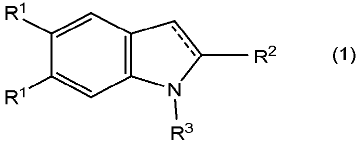 wherein the dashed line represents the presence or absence of a π bond; R¹ represents a hydroxy group or an acetoxy group; R² represents a hydrogen atom or -COOR (wherein R represents a hydrogen atom, a methyl group, or an ethyl group); and R³ represents a hydrogen atom, an acetyl group, a methyl group, or an ethyl group, and
      (b2) an alkali agent
      and having pH of from 8 to 11 to the hair, or
   Step 1B: applying an A2 agent comprising the following component (a1) and (a2):
      (a1) a reducing agent, and
      (a2) 0.03 to 0.3% by mass of a compound of the following formula (1) or a salt thereof: 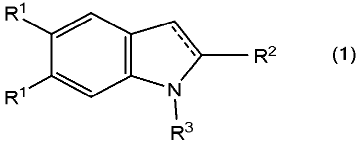 wherein the dashed line represents the presence or absence of a π bond; R¹ represents a hydroxy group or an acetoxy group; R² represents a hydrogen atom or -COOR (wherein R represents a hydrogen atom, a methyl group, or an ethyl group); and R³ represents a hydrogen atom, an acetyl group, a methyl group, or an ethyl group and having pH of from 8 to 11 to hair; and
   Step 2: imparting a shape to the hair,
   the method further comprising in the below order:
      Step 3: applying a C agent comprising an oxidizing agent to the hair; and
      Step 4: rinsing the hair.
<2> The hair-shape-imparting method according to <1>, wherein the component (a1) comprises one or more members selected from the group consisting of thioglycolic acid and a derivative thereof, thiolactic acid and a derivative thereof, cysteine and a derivative thereof, and their salts.
<3> The hair-shape-imparting method according to <1> or <2>, wherein the component (a1) comprises one or more members selected from the group consisting of thioglycolic acid, ammonium thioglycolate, thioglycolic acid glycerin ester, L-cysteine, D-cysteine, N-acetylcysteine, ammonium salts of these cysteines, ethanolamine salts, thioglyceryl alkyl ether, mercaptoethylpropanamide, and mercaptoethylgluconamide, preferably one or more members selected from the group consisting of thioglycolic acid, ammonium thioglycolate, thioglycolic acid glycerin ester, L-cysteine, D-cysteine, N-acetylcysteine, and ammonium salts of these cysteines, more preferably one or more members selected from the group consisting of thioglycolic acid, ammonium thioglycolate, and thioglycolic acid glycerin ester.
<4> The hair-shape-imparting method according to any one of <1> to <3>, wherein a content of the component (a1) reducing agent in the A1 agent or the A2 agent is preferably from 0.1 to 40% by mass, more preferably from 1 to 20% by mass, further more preferably from 2 to 15% by mass, even more preferably from 5 to 10% by mass.
<5> The hair-shape-imparting method according to any one of <1> to <4>, wherein the component (a2) or the component (b1) is one or more members selected from the group consisting of 5,6-dihydroxyindole, 5,6-dihydroxyindole-2-carboxylic acid, methyl 5,6-dihydroxyindole-2-carboxylate, ethyl 5,6-dihydroxyindole-2-carboxylate, N-methyl-5,6-dihydroxyindole, N-methyl-5,6-dihydroxyindole-2-carboxylic acid, N-ethyl-5,6-dihydroxyindole, N-ethyl-5,6-dihydroxyindole-2-carboxylic acid, N-acetyl-5,6-dihydroxyindole, N-acetyl-5,6-dihydroxyindole-2-carboxylic acid, 5-acetoxy-6-hydroxyindole, 5-acetoxy-6-hydroxyindole-2-carboxylic acid, 5,6-dihydroxyindoline, 5,6-dihydroxyindoline-2-carboxylic acid, methyl 5,6-dihydroxyindoline-2-carboxylate, ethyl 5,6-dihydroxyindoline-2-carboxylate, N-methyl-5,6-dihydroxyindoline, N-methyl-5,6-dihydroxyindoline-2-carboxylic acid, N-ethyl-5,6-dihydroxyindoline, N-ethyl-5,6-dihydroxyindoline-2-carboxylic acid, N-acetyl-5,6-dihydroxyindoline, N-acetyl-5,6-dihydroxyindoline-2-carboxylic acid, 5-acetoxy-6-hydroxyindoline, and 5-acetoxy-6-hydroxyindoline-2-carboxylic acid, and hydrochloride, hydrobromide, sulfate, phosphate, acetate, propionate, lactate, and citrate of each of these compounds, preferably hydrobromide of each of these compounds.
<6> The hair-shape-imparting method according to any one of <1> to <5>, wherein the component (a2) or the component (b1) preferably comprises one or more members selected from the group consisting of 5,6-dihydroxyindole, 5,6-dihydroxyindoline, 5,6-dihydroxyindoline-2-carboxylic acid, and their salts, more preferably comprises one or more members selected from the group consisting of 5,6-dihydroxyindole, 5,6-dihydroxyindoline, 5,6-dihydroxyindole-2-carboxylic acid, and 5,6-dihydroxyindoline hydrobromide, further more preferably comprises one or more members selected from the group consisting of 5,6-dihydroxyindole and 5,6-dihydroxyindole-2-carboxylic acid, and even more preferably comprises a mixture of 5,6-dihydroxyindole and 5,6-dihydroxyindole-2-carboxylic acid.
<7> The hair-shape-imparting method according to any one of <1> to <6>, wherein when R² in the formula (1) of the component (a2) or the component (b1) is -COOH, the salt of the compound of the formula (1) is carboxylate thereof (wherein R² is -COO⁻X⁺ (X⁺ is a cation)).
<8> The hair-shape-imparting method according to any one of <1> to <7>, wherein when the component (a2) or the component (b1) comprises 5,6-dihydroxyindole and 5,6-dihydroxyindole-2-carboxylic acid, a molar ratio between the 5,6-dihydroxyindole and the 5,6-dihydroxyindole-2-carboxylic acid is preferably in the range of from 50:50 to 99:1, more preferably in the range of from 80:20 to 99:1, further more preferably in the range of from 85:15 to 95:5.
<9> The hair-shape-imparting method according to any one of <1> to <8>, wherein a content of the component (a2) in the A2 agent or the component (b1) in the B1 agent is from 0.03 to 0.3% by mass, preferably from 0.05 to 0.25% by mass, more preferably from 0.055 to 0.2% by mass, further more preferably from 0.06 to 0.15% by mass, even more preferably from 0.065 to 0.1% by mass.
<10> The hair-shape-imparting method according to any one of <1> to <9>, wherein in the step 1A, a treatment amount ratio between the component (a1) and the component (b1) [(a1)/(b1)] per unit hair dry mass is preferably 45 or more, more preferably 80 or more, further more preferably 150 or more, and preferably 460 or less, more preferably 300 or less, further more preferably 200 or less, and is preferably from 45 to 460, more preferably from 80 to 300, further more preferably from 150 to 200, or in the step 1B, a treatment amount ratio between the component (a1) and the component (a2) [(a1)/(a2)] per unit hair dry mass is preferably 20 or more, more preferably 65 or more, further more preferably 80 or more, and preferably 250 or less, more preferably 150 or less, further more preferably 100 or less, and is preferably from 25 to 250, more preferably from 65 to 150, further more preferably from 80 to 100.
<11> The hair-shape-imparting method according to any one of <1> to <10>, wherein the pH at 25°C of the A1 agent or the A2 agent is from 8 to 11, preferably from 8.5 to 10.5.
<12> The hair-shape-imparting method according to any one of <1> to <11>, wherein the A1 agent or the A2 agent comprises, in addition to the reducing agent and the compound of the formula (1), one or more members selected from the group consisting of an aqueous medium, a pH adjuster, a surfactant, an antioxidant, a viscosity adjuster, silicone, an aromatic alcohol, a coloring component other than the component (a2), a polymer, an oil agent, an anti-dandruff agent, a vitamin preparation, a germicide, an anti-inflammatory agent, an antiseptic, a chelating agent, a humectant, a pearlescent agent, a ceramide, a fragrance, and an ultraviolet absorber.
<13> The hair-shape-imparting method according to any one of <1> to <12>, wherein a formulation of the A1 agent or the A2 agent is a liquid, a foam, a paste, a cream, a solid, or a powder, preferably a liquid, a paste, or a cream.
<14> The hair-shape-imparting method according to any one of <1> to <13>, wherein the hair in applying the A1 agent or the A2 agent thereto is in a dry state or a wet state.
<15> The hair-shape-imparting method according to any one of <1> to <14>, wherein the hair in applying the A1 agent or the A2 agent thereto is in a wet state ascribable to water, and a bath ratio (dry mass of the hair : mass of water) is preferably from 1:0.1 to 1:10, more preferably from 1:0.2 to 1:5, further more preferably from 1:0.3 to 1:3.
<16> The hair-shape-imparting method according to any one of <1> to <15>, wherein a method for applying the A1 agent or the A2 agent to hair is a method of contacting the A1 agent or the A2 agent with the hair, preferably a method of coating the hair with the A1 agent or the A2 agent or a method of immersing the hair in the A1 agent or the A2 agent.
<17> The hair-shape-imparting method according to any one of <1> to <16>, wherein an amount of the A1 agent or the A2 agent applied to the hair is a bath ratio (dry mass of the hair : mass of the A1 agent or the A2 agent) of preferably from 1:0.2 to 1:20, more preferably from 1:0.5 to 1:10, further more preferably from 1:1 to 1:5.
<18> The hair-shape-imparting method according to any one of <1> to <17>, wherein a time for which the A1 agent or the A2 agent is applied to the hair is preferably from 3 to 90 minutes, more preferably from 5 to 60 minutes, further more preferably from 10 to 45 minutes, even more preferably from 10 to 30 minutes.
<19> The hair-shape-imparting method according to any one of <1> to <18>, wherein a temperature in applying the A1 agent or the A2 agent to the hair is preferably from 20 to 40°C, more preferably from 25 to 35°C.
<20> The hair-shape-imparting method according to any one of <1> to <19>, further comprising, after applying the A1 agent or the A2 agent to hair and before applying the B1 agent or the step 2, the step of rinsing the hair.
<21> The hair-shape-imparting method according to any one of <1> to <20>, wherein the component (b2) is one or more members selected from the group consisting of ammonia, alkanolamine, alkylamine, aralkylamine, and an inorganic alkali compound, and the alkanolamine, the alkylamine, or the aralkylamine has preferably 10 or less, more preferably 8 or less carbon atoms.
<22> The hair-shape-imparting method according to any one of <1> to <21>, wherein the component (b2) comprises one or more members selected from the group consisting of ammonia, alkanolamine, alkylamine, aralkylamine, sodium hydroxide, potassium hydroxide, sodium carbonate, sodium bicarbonate, ammonium carbonate, and ammonium bicarbonate.
<23> The hair-shape-imparting method according to any one of <1> to <22>, wherein a content of the component (b2) in the B1 agent is preferably from 0.1 to 10% by mass, more preferably from 0.5 to 8% by mass, further more preferably from 1 to 5% by mass.
<24> The hair-shape-imparting method according to any one of <1> to <23>, wherein the B1 agent further comprises a component (b3) antioxidant.
<25> The hair-shape-imparting method according to <24>, wherein the component (b3) comprises one or more members selected from the group consisting of sulfurous acid, pyrosulfurous acid, hydrogen sulfite, sulfur dioxide, L-ascorbic acid, thioglycolic acid, L-cysteine, N-acetyl-L-cysteine, and their salts, and their derivatives, preferably comprises one or more members selected from the group consisting of L-ascorbic acid, sulfurous acid, pyrosulfurous acid, hydrogen sulfite, and their salts, ascorbic acid glucoside, and sulfur dioxide, more preferably comprises one or more members selected from the group consisting of L-ascorbic acid, sodium sulfite, and sodium ascorbate, and further more preferably comprises one or more members selected from the group consisting of L-ascorbic acid and sodium ascorbate.
<26> The hair-shape-imparting method according to <24> or <25>, wherein a content of the component (b3) in the B1 agent is preferably from 0.05 to 10% by mass, more preferably from 0.1 to 5% by mass, further more preferably from 0.3 to 2% by mass.
<27> The hair-shape-imparting method according to any one of <1> to <26>, wherein the pH at 25°C at the B1 agent is from 8 to 11, more preferably from 8.5 to 10.5.
<28> The hair-shape-imparting method according to any one of <1> to <27>, wherein the B1 agent further comprises a pH adjuster, and the pH adjuster is preferably one or more protonating agents selected from the group consisting of a monobasic acid and a polybasic acid, or one or more protonating agents selected from the group consisting of an organic acid (having 1 or more and 8 or less carbon atoms, except for ascorbic acid) and an inorganic acid, and wherein the protonating agent is more preferably one or more members selected from the group consisting of hydrochloric acid, sulfuric acid, phosphoric acid, formic acid, acetic acid, and citric acid as the protonating agent, further more preferably one or more members selected from the group consisting of phosphoric acid and citric acid.
<29> The hair-shape-imparting method according to <28>, wherein a content of the pH adjuster in the B1 agent is preferably 0.05% by mass or more, more preferably 0.1% by mass or more, further more preferably 0.2% by mass or more, and preferably 5.0% by mass or less, more preferably 4.0% by mass or less, further more preferably 3.5% by mass or less, even more preferably 2.5% by mass or less.
<30> The hair-shape-imparting method according to any one of <1> to <29>, wherein the B1 agent further comprises one or more members selected from the group consisting of an aqueous medium, a higher alcohol, a surfactant, a viscosity adjuster, silicone, an aromatic alcohol, a polymer, an oil agent, an anti-dandruff agent, a vitamin preparation, a germicide, an anti-inflammatory agent, an antiseptic, a chelating agent, a humectant, a pearlescent agent, a ceramide, a fragrance, and an ultraviolet absorber.
<31> The hair-shape-imparting method according to <30>, wherein a cationic surfactant is contained as the surfactant, and the cationic surfactant comprises preferably one or more members selected from the group consisting of monoalkyl trimethylammonium chloride, dialkyl dimethylammonium chloride, monoalkyloxyalkyl trimethylammonium chloride, and monoalkyl trimethylammonium bromide, more preferably one or more members selected from the group consisting of monoalkyl trimethylammonium chloride and monoalkyloxyalkyl trimethylammonium chloride, among them, one or more members selected from the group consisting of behenyl trimethylammonium chloride, stearyl trimethylammonium chloride, cetyl trimethylammonium chloride, lauryl trimethylammonium chloride, dialkyl (C₁₂ to C₁₈) dimethylammonium chloride, and octadecyloxypropyl trimethylammonium chloride.
<32> The hair-shape-imparting method according to <30> or <31>, wherein a content of the cationic surfactant in the B1 agent is preferably 0.1% by mass or more, more preferably 0.5% by mass or more, further more preferably 1.0% by mass or more, even more preferably 2.0% by mass or more, and preferably 10% by mass or less, more preferably 5% by mass or less, further more preferably 4% by mass or less.
<33> The hair-shape-imparting method according to any one of <30> to <32>, wherein the higher alcohol is of the formula R⁴-OH (wherein R⁴ represents a linear or branched hydrocarbon group having 12 or more and 24 or less carbon atoms), and the R⁴ comprises preferably a linear or branched aliphatic hydrocarbon group having 12 or more and 24 or less carbon atoms, more preferably a linear or branched alkyl group having 12 or more and 24 or less carbon atoms or a linear or branched alkenyl group having 12 or more and 24 or less carbon atoms, further more preferably a linear alkyl group having 12 or more and 24 or less carbon atoms or a linear alkenyl group having 12 or more and 24 or less carbon atoms.
<34> The hair-shape-imparting method according to any one of <30> to <33>, wherein the higher alcohol is at least one or more members selected from the group consisting of lauryl alcohol, myristyl alcohol, cetyl alcohol, stearyl alcohol, isostearyl alcohol, arachyl alcohol, behenyl alcohol, carnaubyl alcohol, and oleyl alcohol.
<35> The hair-shape-imparting method according to any one of <30> to <34>, wherein the B1 agent comprises the higher alcohol, and a content of the higher alcohol in the B1 agent is preferably 2% by mass or more, more preferably 3% by mass or more, further more preferably 4% by mass or more, and preferably 10% by mass or less, more preferably 8% by mass or less, further more preferably 7% by mass or less, even more preferably 6% by mass or less.
<36> The hair-shape-imparting method according to any one of <30> to <34>, wherein the B1 agent further comprises a nonionic surfactant and preferably comprises a polyoxyethylene-added nonionic surfactant.
<37> The hair-shape-imparting method according to <36>, wherein the polyoxyethylene-added nonionic surfactant comprises one or more members selected from the group consisting of polyoxyethylene alkyl ether, polyoxyethylene alkenyl ether, polyoxyethylene monofatty acid ester, and polyoxyethylene sorbitan fatty acid ester, and preferably, the alkyl group and the alkenyl group are linear or branched chain, and the fatty acid has a saturated or unsaturated and linear or branched aliphatic chain.
<38> The hair-shape-imparting method according to <37>, wherein the alkyl group, alkenyl group, and the aliphatic chain have preferably 8 or more, more preferably 10 or more, further more preferably 12 or more, and preferably 22 or less, more preferably 20 or less, further more preferably 18 or less carbon atoms.
<39> The hair-shape-imparting method according to any one of <36> to <38>, wherein a content of the nonionic surfactant in the B1 agent is preferably 0.05% by mass or more, more preferably 0.3% by mass or more, further more preferably 0.5% by mass or more, even more preferably 0.7% by mass or more, and preferably 10% by mass or less, more preferably 7% by mass or less, further more preferably 5.0% by mass or less, even more preferably 3.0% or less.
<40> The hair-shape-imparting method according to any one of <1> to <39>, wherein a formulation of the B1 agent is a liquid, a foam, a paste, a cream, a solid, or a powder, preferably a liquid, a paste, or a cream.
<41> The hair-shape-imparting method according to any one of <1> to <40>, wherein the hair in applying the B1 agent thereto is in a dry state or a wet state.
<42> The hair-shape-imparting method according to any one of <1> to <41>, wherein a method for applying the B1 agent to hair is a method of contacting the B1 agent with the hair, preferably a method of coating the hair with the B1 agent or a method of immersing the hair in the B1 agent.
<43> The hair-shape-imparting method according to any one of <1> to <42>, wherein an amount of the B1 agent applied to the hair is a bath ratio (dry mass of the hair : mass of the B1 agent) of preferably from 1:0.1 to 1:20, more preferably from 1:0.3 to 1:10, further more preferably from 1:0.5 to 1:5.
<44> The hair-shape-imparting method according to any one of <1> to <43>, wherein a time for which the B1 agent is applied to the hair is preferably 1 to 60 minutes, more preferably 3 to 30 minutes, further more preferably 4 to 15 minutes.
<45> The hair-shape-imparting method according to any one of <1> to <44>, further comprising applying the B1 agent to the hair and before the step 2, the step of rinsing the hair.
<46> The hair-shape-imparting method according to <45>, further comprising, after the step of rinsing the hair, a drying step of drying the hair, wherein preferably one or more drying treatments selected from the group consisting of towel drying, blow (cold air or hot air) drying, and air drying is performed, and more preferably two or more of the drying treatments are performed in combination.
<47> The hair-shape-imparting method according to any one of <1> to <46>, wherein the step 2 is the step of drying the hair and heating the hair with a hair iron to impart a shape to the hair.
<48> The hair-shape-imparting method according to <47>, wherein a heating temperature with the hair iron is preferably from 100 to 220°C, more preferably from 120 to 220°C, further more preferably from 140 to 210°C.
<49> The hair-shape-imparting method according to any one of <1> to <48>, wherein the oxidizing agent comprises one or more members selected from the group consisting of potassium bromate, sodium bromate, sodium perborate, and hydrogen peroxide, preferably comprises one or more members selected from the group consisting of sodium bromate and hydrogen peroxide, and is more preferably sodium bromate or hydrogen peroxide.
<50> The hair-shape-imparting method according to any one of <1> to <49>, wherein a content of the oxidizing agent is preferably from 1 to 20% by mass, more preferably from 2 to 10% by mass, further more preferably from 3 to 8% by mass.
<51> The hair-shape-imparting method according to any one of <1> to <50>, wherein the C agent comprises, in addition to the oxidizing agent, one or more members selected from the group consisting of an aqueous medium, a pH adjuster, a surfactant, a viscosity adjuster, silicone, an aromatic alcohol, a polymer, an oil agent, an anti-dandruff agent, a vitamin preparation, a germicide, an anti-inflammatory agent, an antiseptic, a chelating agent, a humectant, a pearlescent agent, a ceramide, a fragrance, and an ultraviolet absorber.
<52> The hair-shape-imparting method according to any one of <1> to <51>, wherein the hair in applying the C agent thereto is in a dry state or a wet state.
<53> The hair-shape-imparting method according to any one of <1> to <52>, wherein a method for applying the C agent to hair is a method of contacting the C agent with the hair, preferably a method of coating the hair with the C agent or a method of immersing the hair in the C agent.
<54> The hair-shape-imparting method according to any one of <1> to <53>, wherein an amount of the C agent applied to the hair is a bath ratio (dry mass of the hair : mass of the C agent) of preferably from 1:0.1 to 1:20, more preferably from 1:0.5 to 1:10, further more preferably from 1:1 to 1:5.
<55> The hair-shape-imparting method according to any one of <1> to <54>, wherein a time for which the C agent is applied to the hair is preferably from 3 to 90 minutes, more preferably from 5 to 60 minutes, further more preferably from 10 to 45 minutes, even more preferably from 10 to 30 minutes.
<56> The hair-shape-imparting method according to any one of <1> to <55>, wherein the step 4 is the step of, after applying the C agent to the hair, rinsing the hair with water.
<57> The hair-shape-imparting method according to any one of <1> to <56>, wherein the shape to be imparted to the hair is a straight or curled shape.
<58> A kit for imparting a hair shape, comprising:
   an A1 agent comprising the following component (a1):
      (a1) a reducing agent
      and having pH of from 8 to 11;
   a B1 agent comprising the following component (b1) and component (b2):
      (b1) 0.03 to 0.3% by mass of a compound of the following formula (1) or a salt thereof: 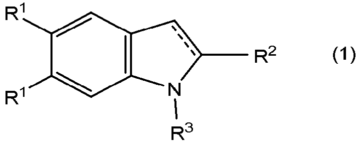 wherein the dashed line represents the presence or absence of a π bond; R¹ represents a hydroxy group or an acetoxy group; R² represents a hydrogen atom or -COOR (wherein R represents a hydrogen atom, a methyl group, or an ethyl group); and R³ represents a hydrogen atom, an acetyl group, a methyl group, or an ethyl group, and
      (b2) an alkali agent
      and having pH of from 8 to 11; and
   a C agent comprising an oxidizing agent.
<59> The kit for imparting a hair shape according to <58>, wherein the A1 agent has a configuration according to any one of <2> to <4> and <11> to <13>.
<60> The kit for imparting a hair shape according to <58> or <59>, wherein the B1 agent has a configuration according to any one of <5> to <10> and <21> to <40>.
<61> The kit for imparting a hair shape according to any one of <58> to <60>, wherein the C agent has a configuration according to any one of <49> to <51>.
<62> A kit for imparting a hair shape, comprising:
   an A2 agent comprising the following components (a1) and (a2):
      (a1) a reducing agent, and
      (a2) 0.03 to 0.3% by mass of a compound of the following formula (1) or a salt thereof: 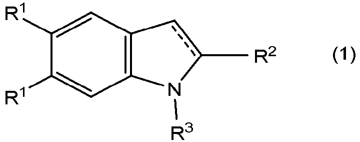 wherein the dashed line represents the presence or absence of a π bond; R¹ represents a hydroxy group or an acetoxy group; R² represents a hydrogen atom or -COOR (wherein R represents a hydrogen atom, a methyl group, or an ethyl group); and R³ represents a hydrogen atom, an acetyl group, a methyl group, or an ethyl group and having pH of from 8 to 11; and
   a C agent comprising an oxidizing agent.
<63> The kit for imparting a hair shape according to <62>, wherein the A2 agent has a configuration according to any one of <2> to <13>.
<64> The kit for imparting a hair shape according to <62> or <63>, wherein the C agent has a configuration according to any one of <49> to <51>.

### Examples

Hereinafter, the present invention will be described with reference to Examples. However, the present invention is not limited by the scope of Examples. In the present Examples, various measurements and evaluations were performed by the following methods.

### <pH measurement>

pH at 25°C of an A agent and a B1 agent was measured using a pH meter (F-51, manufactured by HORIBA, Ltd.).

### <Hair straightening>

Change in shape of a tress after a hair treatment mentioned later was evaluated by five expert panelists, and the number of panelists who evaluated the hair as "being straightened by the treatment" was used as an evaluation value. A higher evaluation value means a better hair shape-imparting effect.

### <Manageable feeling>

Manageable feeling of hair after a hair treatment mentioned later was evaluated as manageable feeling of a tress treated under each condition and dried, by five expert panelists, and the number of panelists who made evaluation as "neat and manageable hair grain" from the root toward the tip of the hair was used as an evaluation value. A higher evaluation value means better manageable feeling.

### <Supple and resilient feeling>

Supple and resilient feeling of hair after a hair treatment mentioned later was evaluated as supple and resilient feeling of a tress treated under each condition and dried, by five expert panelists, and the number of panelists who evaluated the hair as "having supple and resilient feeling as compared with an untreated tress" when touching the hair was used as an evaluation value. A higher evaluation value means that neither suppleness nor resilience is impaired.

### <Change in L value after treatment>

An L value of a tress was measured in order to evaluate change in hair lightness between before and after a treatment. In the measurement of the L value, a lightness (L*) of a tress before a treatment or a tress after a hair treatment mentioned later was measured at six locations per strand of hair using a colorimeter (CR-400 manufactured by Konica Minolta, Inc.), and an average value was determined. A smaller absolute value of change in L value means that a hair color is not changed. In this context, L* refers to a lightness L* defined by the CIE1976 L*a*b* color system.

### <Apparent color and lightness>

Change in color and lightness of hair after a treatment was evaluated as a color and lightness of a tress subjected to a hair treatment mentioned later and dried, by five expert panelists, and the number of panelists who made evaluation as "no change in color and lightness as compared with an untreated tress" was used as an evaluation value. A higher evaluation value means that a hair color is not changed.

### <Hair wash fastness>

Hair wash fastness of hair after a treatment was evaluated as change in shape of a tress subjected to a hair treatment mentioned later and then a hair wash treatment with a plain shampoo mentioned later 7 times, by five expert panelists, and the number of panelists who evaluated the "hair shape as being maintained after the hair wash treatment" was used as an evaluation value. A higher evaluation value means that an imparted hair shape has hair wash fastness.

### <Preparing tress for evaluation>

Black curly hair of Japanese (not for sale) having no chemical treatment history was used, and a tress was prepared such that a length when the hair was straightened by the application of a slight load thereto was from 30 to 35 cm and a mass was from 10 to 15 g. This tress was cleansed with a shampoo having the following prescription, rinsed, then rid of excess water by wiping with a towel, and naturally dried for 12 hours under laboratory conditions, and the resulting tress was used in evaluations.

| (Plain shampoo) | (% by mass) |
|---|---|
| Sodium polyoxyethylene lauryl ether sulfate (EMAL E-27C (amount of active ingredient: 27% by mass), by mass), manufactured by Kao Corp.) | 57.4 |
| Lauramide DEA (AMINON L-02, manufactured by Kao Corp.) | 1.5 |
| EDTA-2Na (FROST DS, manufactured by Daiichi Pure Chemicals Co., Ltd.) | 0.3 |
| Phosphoric acid (for pH adjustment to 7.0) | |
| Sodium benzoate | 0.5 |
| Purified water | balance |
| Total | 100 |

### <Hair treatment>

### Examples 1 to 5 and Comparative Examples 1 and 2 (Preparing A agent, B1 agent, and C agent and coating)

The components of each part shown in Table 1 were blended at the blending ratio described in in Table 1, and mixed until homogeneous to prepare an A agent, a B1 agent, and a C agent.

The tress for evaluation in the state shown in Table 1 was coated with the A agent at the bath ratio (dry mass of the hair : mass of the A agent) shown in Table 1, kept for the application time shown in Table 1, and rinsed; and as for the tress in each example other than Example 4 and Comparative Example 1, the tress in the state shown in Table 1 was coated with the B1 agent at the bath ratio (dry mass of the hair : mass of the B1 agent) shown in Table 1, kept for the application time shown in Table 1, and rinsed (step 1A or step 1B). The tress was blow-dried with a dryer and heated under the conditions shown in Table 1 using a hair iron to impart a straight shape thereto (step 2). Then, the tress was coated with the C agent at the bath ratio (dry mass of the hair : mass of the C agent) shown in Table 1, kept for the application time shown in Table 1 (step 3), and rinsed (step 4) to perform a hair treatment. The tress thus treated was evaluated by the methods described above. The results are shown in Table 1.

All the amounts of components blended (% by mass) described in Table 1 are the amount of an active ingredient, and the total was 100% by mass for each of the A agent and the B1 agent.

### [Table 1]

**Table 1**

| | | Example | | | | | Comparative Example | |
|---|---|---|---|---|---|---|---|---|
| | | 1 | 2 | 3 | 4 | 5 | 1 | 2 |
| A agent | | A1 agent | A1 agent | A1 agent | A2 agent | A1 agent | A1 agent | A1 agent |
| | (a1) Ammonium thioglycolate | 6.8 | 6.8 | 6.8 | 6.8 | 6.8 | 6.8 | 6.8 |
| | (a2) 5,6-Dihydroxyindole/5,6-dihydroxyindole-2-carboxylic acid mixture | | | | 0.075 | | | |
| | 75% phosphoric acid | q.s. | q.s. | q.s. | q.s. | q.s. | q.s. | q.s. |
| | Water | Balance | Balance | Balance | Balance | Balance | Balance | Balance |
| | pH | 9 | 9 | 9 | 9 | 9 | 9 | 9 |
| Application conditions of A agent | State of hair tress before application | Wet hair^{*6} | Wet hair^{*6} | Wet hair^{*6} | Wet hair^{*6} | Wet hair^{*6} | Wet hair^{*6} | Wet hair^{*6} |
| | Bath ratio (hair : A agent) | 1:2 | 1:2 | 1:2 | 1:2 | 1:2 | 1:2 | 1:2 |
| | Application time (min) | 15 | 15 | 15 | 15 | 15 | 15 | 15 |
| | Treatment dosage form | Cream formulation | Cream formulation | Cream formulation | | Aqueous formulation | | Cream formulation |
| | (b1) 5,6-Dihydroxyindole/5,6-dihydroxyindole-2-carboxylic acid mixture | 0.075 | 0.05 | 0.25 | | 0.075 | | 0.5 |
| | (b2) Monoethanolamine | 3 | 3 | 3 | | 3 | | 3 |
| | (b3) L-Ascorbic acid | 0.6 | 0.6 | 0.6 | | 0.6 | | 0.6 |
| B1 agent | Stearyl trimethylammonium chloride (28%)^{*1} | 3.2 | 3.2 | 3.2 | | | | 3.2 |
| | Polyoxyethylene (40) cetyl ether^{*2} | 1.1 | 1.1 | 1.1 | | | | 1.1 |
| | Polyoxyethylene (2) cetyl ether^{*3} | 0.5 | 0.5 | 0.5 | | | | 0.5 |
| | Stearyl alcohol^{*4} | 2.2 | 2.2 | 2.2 | | | | 2.2 |
| | Behenyl alcohol^{*5} | 2.8 | 2.8 | 2.8 | | | | 2.8 |
| | 75% phosphoric acid | q.s. | q.s. | q.s. | | q.s. | | q.s. |
| | Water | Balance | Balance | Balance | | Balance | | Balance |
| | pH | 10.2 | 10.2 | 10.2 | | 10.2 | | 10.2 |
| Application conditions of B1 agent | State of hair tress before application | Wet hair^{*6} | Wet hair^{*6} | Wet hair^{*6} | | Wet hair^{*6} | | Wet hair^{*6} |
| | Bath ratio (hair : B1 agent) | 1:1 | 1:1 | 1:1 | | 1:1 | | 1:1 |
| | Application time (min) | 5 | 5 | 5 | | 5 | | 5 |
| Presence or absence of heating step | 200°C, 10 sec (hair iron) | Present | Present | Present | Present | Present | Present | Present |
| C agent | Sodium bromate | 6 | 6 | 6 | 6 | 6 | 6 | 6 |
| | Water | Balance | Balance | Balance | Balance | Balance | Balance | Balance |
| Application conditions of C agent | Bath ratio (hair : C agent) | 1:2 | 1:2 | 1:2 | 1:2 | 1:2 | 1:2 | 1:2 |
| | Application time (min) | 15 | 15 | 15 | 15 | 15 | 15 | 15 |
| Ratio of treatment amount [(a1)/(a2)] or [(a1)/(b1)] | | 181.3 | 272.0 | 54.4 | 90.7 | 181.3 | - | 27.2 |
| Evaluation of hair after treatment | Hair straightening | 5 | 5 | 5 | 5 | 5 | 5 | 5 |
| | Manageable feeling | 5 | 4 | 5 | 3 | 3 | 2 | 5 |
| | Supple and resilient feeling | 5 | 3 | 5 | 4 | 5 | 0 | 5 |
| | Change in L value after treatment | -0.27 | 0.22 | -0.44 | -0.31 | -0.22 | 0.38 | 2.03 |
| | Apparent color and lightness | 5 | 3 | 3 | 4 | 5 | 1 | 0 |
| | Hair wash fastness | 5 | 5 | 5 | 5 | 5 | 5 | 5 |

| | | | | | | | | |
|---|---|---|---|---|---|---|---|---|
| *6: Hair tress in a state having a bath ratio (hair tress : water) of 1:0.5 | | | | | | | | |

The components described in the table are as follows.
(a2) and (b1): 5,6-Dihydroxyindole/5,6-dihydroxyindole-2-carboxylic acid mixture: produced by the method described in JP-B-5570161 for use. The molar ratio between the 5,6-dihydroxyindole and the 5,6-dihydroxyindole-2-carboxylic acid was 90:10. The components (a2) and (b1) were prepared as a solution containing 1% by mass thereof in H₂O/ethanol = 4/1 (wt/wt), which was blended with the A agent or the B1 agent.
*1: QUARTAMIN 86W (stearyl trimethylammonium chloride, amount of active ingredient: 28% by mass, Kao Corp.)
*2: NIKKOL BC-40TX (Nikko Chemicals Co., Ltd.)
*3: NIKKOL BC-2 (Nikko Chemicals Co., Ltd.)
*4: KALCOL 8098 (Kao Corp.)
*5: KALCOL 220-80 (Kao Corp.)

The B1 agent of Example 5 was an aqueous composition (aqueous preparation) which was blended with neither the cationic surfactant nor the higher alcohol.

As is evident from Table 1, the method of the present invention can impart a hair shape having sufficient hair wash fastness without changing a hair color, impairs neither hair suppleness nor resilience, and offers excellent manageable feeling.

### Industrial Applicability

The present invention can provide a hair-shape-imparting method which can impart a hair shape having sufficient hair wash fastness without changing a hair color, impairs neither hair suppleness nor resilience, and offers excellent manageable feeling.

## Claims

1. A hair-shape-imparting method comprising in the below order:
Step 1A: applying an A1 agent comprising the following component (a1):
(a1) a reducing agent
and having pH of from 8 to 11 to hair, and
then applying a B1 agent comprising the following component (b1) and component (b2):
(b1) 0.03 to 0.3% by mass of a compound of the following formula (1) or a salt thereof: 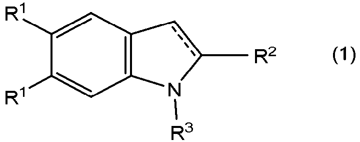 wherein the dashed line represents the presence or absence of a π bond; R¹ represents a hydroxy group or an acetoxy group; R² represents a hydrogen atom or -COOR, wherein R represents a hydrogen atom, a methyl group, or an ethyl group; and R³ represents a hydrogen atom, an acetyl group, a methyl group, or an ethyl group, and
(b2) an alkali agent
and having pH of from 8 to 11 to the hair, or
Step 1B: applying an A2 agent comprising the following component (a1) and (a2):
(a1) a reducing agent, and
(a2) 0.03 to 0.3% by mass of a compound of the following formula (1) or a salt thereof: 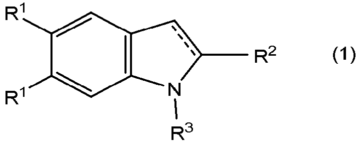 wherein the dashed line represents the presence or absence of a π bond; R¹ represents a hydroxy group or an acetoxy group; R² represents a hydrogen atom or -COOR, wherein R represents a hydrogen atom, a methyl group, or an ethyl group; and R³ represents a hydrogen atom, an acetyl group, a methyl group, or an ethyl group and having pH of from 8 to 11 to hair; and
Step 2: imparting a shape to the hair,
the method further comprising in the below order:
Step 3: applying a C agent comprising an oxidizing agent to the hair; and
Step 4: rinsing the hair.

2. The hair-shape-imparting method according to claim 1, wherein the step 2 is the step of drying the hair and heating the hair with a hair iron to impart a shape to the hair.

3. The hair-shape-imparting method according to claim 1 or 2, wherein the shape to be imparted to the hair is a straight or curled shape.

4. The hair-shape-imparting method according to any of claims 1 to 3, wherein the component (a1) comprises one or more members selected from the group consisting of thioglycolic acid, ammonium thioglycolate, thioglycolic acid glycerin ester, L-cysteine, D-cysteine, N-acetylcysteine, and ammonium salts of these cysteines.

5. The hair-shape-imparting method according to any of claims 1 to 4, wherein the component (b2) comprises one or more members selected from the group consisting of ammonia, alkanolamine, alkylamine, aralkylamine, sodium hydroxide, potassium hydroxide, sodium carbonate, sodium bicarbonate, ammonium carbonate, and ammonium bicarbonate.

6. The hair-shape-imparting method according to any of claims 1 to 5, wherein the oxidizing agent is sodium bromate or hydrogen peroxide.

7. A kit for imparting a hair shape, comprising:
an A1 agent comprising the following component (a1):
(a1) a reducing agent
and having pH of from 8 to 11;
a B1 agent comprising the following component (b1) and component (b2):
(b1) 0.03 to 0.3% by mass of a compound of the following formula (1) or a salt thereof: 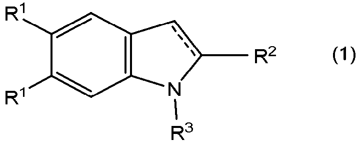 wherein the dashed line represents the presence or absence of a π bond; R¹ represents a hydroxy group or an acetoxy group; R² represents a hydrogen atom or -COOR, wherein R represents a hydrogen atom, a methyl group, or an ethyl group; and R³ represents a hydrogen atom, an acetyl group, a methyl group, or an ethyl group, and
(b2) an alkali agent
and having pH of from 8 to 11; and
a C agent comprising an oxidizing agent.

8. A kit for imparting a hair shape, comprising:
an A2 agent comprising the following components (a1) and (a2):
(a1) a reducing agent, and
(a2) 0.03 to 0.3% by mass of a compound of the following formula (1) or a salt thereof: 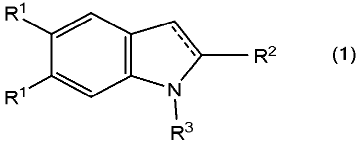 wherein the dashed line represents the presence or absence of a π bond; R¹ represents a hydroxy group or an acetoxy group; R² represents a hydrogen atom or -COOR, wherein R represents a hydrogen atom, a methyl group, or an ethyl group; and R³ represents a hydrogen atom, an acetyl group, a methyl group, or an ethyl group and having pH of from 8 to 11; and
a C agent comprising an oxidizing agent.
